# EUROPEAN PATENT APPLICATION

(11) **EP 4 768 018 A1**
(43) Date of publication of application: **01.07.2026**
(21) Application number: 24855870.2
(22) Date of filing: 22.08.2024
(51) Int. Cl.: A61K 9/06, A61K 31/519, C07D 487/04, A61P 19/02

(54) **TOPICAL GEL FOR JAK INHIBITOR AND USE THEREOF**

(30) Priority: 22.08.2023 CN 202311062820
(71) Applicant: Prime Gene Therapeutics Co., Ltd., Beijing 100176 (CN)
(72) Inventor: LI, Ming, Beijing 100176 (CN); YANG, Xiupin, Beijing 100176 (CN); HUANG, Na, Beijing 100176 (CN); LIU, Yan, Beijing 100176 (CN); DU, Daniel Yunlong, Beijing 100176 (CN)
(74) Representative: Høiberg P/S
(86) International application number: PCT/CN2024/113878
(87) International publication number: WO 2025/040148

(57) **Abstract**

The present disclosure provides a topical gel formulation comprising 2-{3-[3-amino-4-(7H-pyrrolo[2,3-d]pyrimidin-4-yl)-1H-pyrazol-1-yl]-1-(isopropylsulfonyl)azetidin-3-yl}acetonitrile or its crystal form, a gel matrix, and other pharmaceutical excipients.

## Description

### Technical Field

The present disclosure relates to the field of pharmaceutical formulations, and more particularly to a topical gel formulation of a JAK inhibitor and use thereof.

### Background Art

2-{ 3-[3-amino-4-(7H-pyrrolo[2,3-d]pyrimidin-4-yl)-1H-pyrazol-1-yl]-1-(isopropylsulfonyl)azetidin-3-yl}acetonitrile (Compound A) represented by formula A 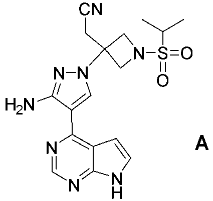 is a small-molecule non-receptor tyrosine kinase inhibitor of the JAK class. The JAK-STAT signaling pathway is closely related to inflammatory cytokines and tumors, and is widely involved in important biological processes such as cell proliferation, differentiation, metastasis, apoptosis, regulation of immune responses, and cellular homeostasis in human health and disease processes.

As a topical formulation for local administration and local action, the active ingredient can penetrate the skin and maintain long-term retention in the skin to exert its effect, while minimal systemic exposure is a major advantage of such topical formulations. In topical formulations, gel formulations are mostly aqueous-based, which are non-greasy and easy to clean after contact with clothing during clinical use, providing good clinical compliance.

### Summary

The present disclosure provides a topical gel formulation and a preparation method thereof. This gel formulation is non-irritating, uniform and delicate, easy to apply, and has a rapid onset of action. It can quickly penetrate the skin and remain in the local skin to exert its pharmacological effect, making it an excellent topical delivery formulation.

According to one aspect, the present disclosure provides a topical gel formulation comprising:
(a) 2-{3-[3-amino-4-(7H-pyrrolo[2,3-d]pyrimidin-4-yl)-1H-pyrazol-1-yl]-1-(isopropylsulfonyl)azetidin-3-yl}acetonitrile, its hydrate, its pharmaceutically acceptable salt, or its crystal form;
(b) a gel matrix;
(c) propylene glycol in an amount of 5% to 30% by total weight of the topical gel formulation; and
(d) polyethylene glycol in an amount of 2% to 20% by total weight of the topical gel formulation.

According to some embodiments, component (a) in the topical gel formulation of the present application is 2-{3-[3-amino-4-(7H-pyrrolo[2,3-d]pyrimidin-4-yl)-1H-pyrazol-1-yl]-1-(isopropylsulfonyl)azetidin-3-yl}acetonitrile hemihydrate.

According to some embodiments, the gel matrix in the topical gel formulation of the present application can be carbomer. Carbomer is in an amount of, for example, 0.8% to 1.5%, and further for example, 1% by total weight of the topical gel formulation.

According to some embodiments, the average molecular weight of the polyethylene glycol in the topical gel formulation of the present disclosure can be 200 to 600, such as polyethylene glycol 200, polyethylene glycol 400, or polyethylene glycol 600. The amount of polyethylene glycol in the topical gel formulation is, for example, 5% by total weight of the topical gel formulation.

According to some embodiments, the amount of propylene glycol in the topical gel formulation of the present disclosure is about 15% (w/w) by total weight of the topical gel formulation.

According to another aspect, the present disclosure provides a topical gel formulation comprising:
(a) 2-{3-[3-amino-4-(7H-pyrrolo[2,3-d]pyrimidin-4-yl)-1H-pyrazol-1-yl]-1-(isopropylsulfonyl)azetidin-3-yl}acetonitrile hemihydrate crystal form, wherein the characteristic peaks in the powder X-ray diffraction pattern of the crystal form have 2θ values of 7.93, 9.36, 11.29, 14.95, 20.96, 21.36, 21.77, 22.12, and 22.79, with a measurement error of ±0.2 degrees.

According to some embodiments, the topical gel formulation of the present disclosure further comprises:
(b) a gel matrix; and
(c) propylene glycol in an amount of 5% to 30% by total weight of the topical gel formulation.

According to some embodiments, the topical gel formulation of the present disclosure further comprises polyethylene glycol. The average molecular weight of the polyethylene glycol in the topical gel formulation of the present disclosure may be 200 to 600, such as polyethylene glycol 200, polyethylene glycol 400, or polyethylene glycol 600.

According to some embodiments, the amount of polyethylene glycol in the topical gel formulation of the present disclosure is 2% to 20%, for example, 5% by total weight of the topical gel formulation.

According to some embodiments, the gel matrix in the topical gel formulation of the present disclosure may be carbomer. The amount of gel matrix (such as carbomer) is, for example, 0.8% to 1.5%, such as 1%, by total weight of the topical gel formulation.

According to some embodiments, the amount of propylene glycol in the topical gel formulation of the present disclosure is about 15% (w/w) by total weight of the topical gel formulation.

According to another aspect, the present disclosure provides a topical gel formulation comprising:
(a) 2-{3-[3-amino-4-(7H-pyrrolo[2,3-d]pyrimidin-4-yl)-1H-pyrazol-1-yl]-1-(isopropylsulfonyl)azetidin-3-yl}acetonitrile hemihydrate crystal form, wherein the characteristic peaks in the powder X-ray diffraction pattern of the crystal form have 2θ values of 7.93, 9.36, 11.29, 14.95, 20.96, 21.36, 21.77, 22.12, and 22.79, with a measurement error of ±0.2 degrees; and
(b) a gel matrix, such as carbomer, in an amount of 0.8% to 1.5%, for example, 1% by total weight of the topical gel formulation.

According to some embodiments, the topical gel formulation of the present disclosure further comprises polyethylene glycol and propylene glycol.

According to some embodiments, the propylene glycol in the topical gel formulation of the present disclosure is in an amount of 5% to 30%, such as 15% by total weight of the topical gel formulation.

According to some embodiments, the polyethylene glycol in the topical gel formulation of the present disclosure is 2% to 20%, such as 5% by total weight of the topical gel formulation.

According to some embodiments, the average molecular weight of the polyethylene glycol in the topical gel formulation of the present disclosure may be 200 to 600, such as polyethylene glycol 200, polyethylene glycol 400, or polyethylene glycol 600.

According to some embodiments, the topical gel formulation of the present disclosure may further comprise one or more of the following pharmaceutical excipients: antimicrobial agents, antioxidants, chelating agents, and pH adjusters.

In the present disclosure, the gel matrix refers to a class of substances that can form colloids which have a spatial network structure.

In the topical gel formulation of the present disclosure, the gel matrix includes but is not limited to one or more of hydroxypropyl methylcellulose, methylcellulose, ethylcellulose, and carbomer; preferably carbomer; more preferably one or more of carbomer homopolymer Type A, carbomer homopolymer Type B, and carbomer homopolymer Type C.

In some embodiments, the polyethylene glycol may be selected from one or more of polyethylene glycol 200, polyethylene glycol 300, and polyethylene glycol 400; preferably polyethylene glycol 400.

In some embodiments, the chelating agent, if included in the topical gel formulation, may be selected from one or more of disodium edetate and calcium sodium edetate; preferably disodium edetate.

In some embodiments, the antimicrobial agent, if included in the topical gel formulation, may be selected from one or more of phenoxyethanol, potassium sorbate, benzyl alcohol, methylparaben, propylparaben, sodium methylparaben, sodium propylparaben, chlorhexidine digluconate, chloroxylenol, chlorphenesin, dehydroacetic acid, diazolidinyl urea, DMDM hydantoin, ethylparaben, butyl iodopropynyl carbamate, methylisothiazolinone, propylparaben, phenoxyethanol, phenoxyisopropanol, polyaminopropyl biguanide, benzoate, and salicylic acid; preferably phenoxyethanol, potassium sorbate, benzyl alcohol, methylparaben, propylparaben, sodium methylparaben, and sodium propylparaben; more preferably methylparaben, propylparaben, sodium methylparaben, and sodium propylparaben.

In some embodiments, the topical gel formulation further comprises an antioxidant, which can be selected from one or more of butylated hydroxytoluene, butylated hydroxyanisole, propyl gallate, vitamin C, sodium bisulfite, sodium metabisulfite, and cysteine hydrochloride; preferably butylated hydroxyanisole and propyl gallate.

In some embodiments, the topical gel formulation further comprises a pH adjuster, which can be selected from one or more of sodium hydroxide, potassium hydroxide, triethanolamine, and hydrochloric acid; preferably triethanolamine or sodium hydroxide.

{3-[3-amino-4-(7H-pyrrolo[2,3-d]pyrimidin-4-yl)-1H-pyrazol-1-yl]-1-(isopropylsulfonyl)azetidin-3-yl } acetonitrile (Compound A) has multiple crystal forms, including Crystal Form I, Crystal Form IIa, Crystal Form IIb, and Crystal Form III. Crystal Form I and Crystal Form IIa are anhydrous forms, Crystal Form IIb is a hemihydrate, and Crystal Form III is amorphous. The X-ray powder diffraction data and patterns for different crystal forms are as follows:
Crystal Form IIa has a powder X-ray diffraction pattern with characteristic peaks at 2θ = 7.61, 9.28, 12.44, 12.96, 14.28, 15.30, 17.56, 18.23, 21.05, 21.47, 22.28, 23.45, 24.87, and 26.03.

Crystal Form IIb has a powder X-ray diffraction pattern with characteristic peaks at 2θ = 7.93, 9.36, 11.29, 14.95, 20.96, 21.36, 21.77, 22.12, and 22.79.

Crystal Form IIb has more characteristic peaks at 2θ = 7.93, 9.36, 11.29, 14.69, 14.95, 15.13, 15.95, 19.93, 20.15, 20.96, 21.36, 21.77, 22.12, 22.79, 23.54, 24.14, 26.38, 28.74, and 29.15.

Crystal Form I has a powder X-ray diffraction pattern with characteristic peaks at 2θ = 6.48, 7.68, 9.90, 13.01, 14.81, 15.38, 16.43, 16.87, 19.61, 20.68, 23.47, 26.29, and 32.23.

**Table 1 XRPD Peaks for Crystal Form IIa**

| **2θ** | **d- (Å)** | **Relative Intensity (%)** |
|---|---|---|
| 7.61 | 11.59 | 100.0 |
| 9.28 | 9.51 | 7.8 |
| 12.44 | 7.10 | 4.5 |
| 12.96 | 6.82 | 8.8 |
| 14.28 | 6.19 | 8.1 |
| 15.30 | 5.78 | 15.0 |
| 17.56 | 5.04 | 21.4 |
| 18.23 | 4.86 | 37.6 |
| 21.05 | 4.51 | 6.7 |
| 21.47 | 4.13 | 22.7 |
| 22.28 | 3.98 | 6.9 |
| 23.45 | 3.79 | 9.9 |
| 24.87 | 3.57 | 4.0 |
| 26.03 | 3.41 | 6.6 |

**Table 2 XRPD Peaks for Crystal Form IIb**

| **2θ** | **d- (Å)** | **Relative Intensity (%)** |
|---|---|---|
| 7.93 | 11.13 | 100.0 |
| 9.36 | 9.43 | 4.1 |
| 11.29 | 7.82 | 2.9 |
| 14.69 | 6.02 | 18.9 |
| 14.95 | 5.91 | 13.1 |
| 15.13 | 5.85 | 4.8 |
| 15.95 | 5.55 | 13.3 |
| 19.93 | 4.44 | 9.7 |
| 20.15 | 4.40 | 6.9 |
| 20.96 | 4.23 | 4.9 |
| 21.36 | 4.15 | 12.3 |
| 21.77 | 4.07 | 10.3 |
| 22.12 | 4.01 | 5.1 |
| 22.79 | 3.89 | 11.7 |
| 23.54 | 3.77 | 5.4 |
| 24.14 | 3.68 | 7.9 |
| 26.38 | 3.37 | 2.6 |
| 28.74 | 3.10 | 2.1 |
| 29.15 | 3.06 | 3.7 |
| 31.69 | 2.82 | 2.8 |

**Table 3 XRPD Peaks for Crystal Form I**

| **2θ** | **d- (Å)** | **Relative Intensity** |
|---|---|---|
| 6.48 | 13.62 | 100.0 |
| 7.68 | 11.49 | 2.7 |
| 9.90 | 8.92 | 2.8 |
| 13.01 | 6.79 | 47.7 |
| 14.81 | 5.97 | 4.5 |
| 15.38 | 5.75 | 8.9 |
| 16.43 | 5.38 | 9.4 |
| 16.87 | 5.25 | 5.5 |
| 19.61 | 4.52 | 58.0 |
| 20.68 | 4.29 | 8.2 |
| 23.47 | 3.78 | 17.6 |
| 26.29 | 3.38 | 11.4 |
| 32.23 | 2.77 | 4.7 |

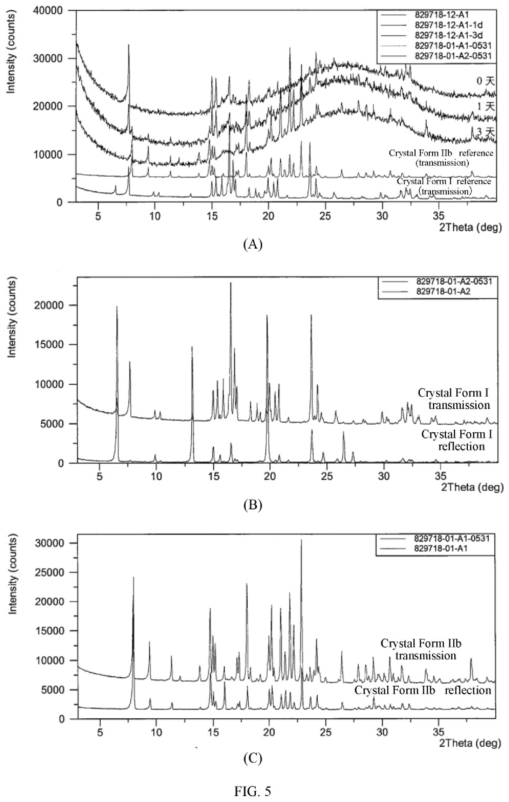

Crystal Form IIa converts to Crystal Form IIb within 1 hour at room temperature (58.6% RH). During the preparation of topical gel formulation, Crystal Form I and Crystal Form IIb were respectively used as active pharmaceutical ingredients. Surprisingly, after 3 days, the final gel formulations were found to contain the active pharmaceutical ingredient exclusively in form IIb, as shown in FIG. 5 to FIG. 6.

### Description of Drawings

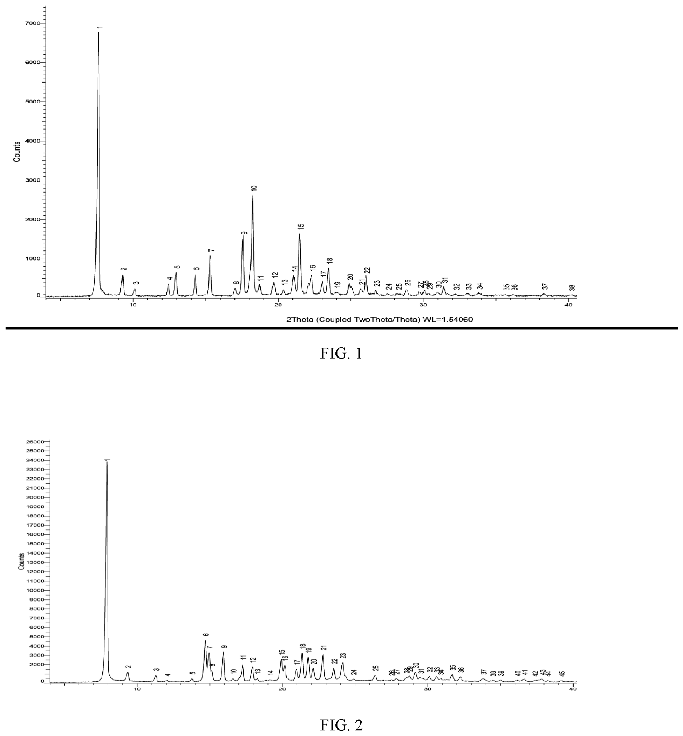

FIG. 1 shows XRPD pattern of Crystal Form IIa.
FIG. 2 shows XRPD pattern of Crystal Form IIb.

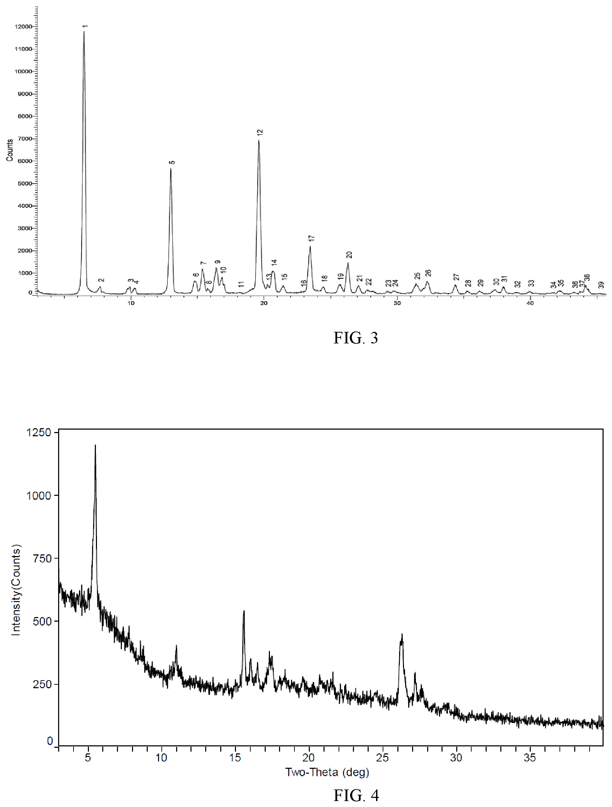

FIG. 3 shows XRPD pattern of Crystal Form I.
FIG. 4 shows XRPD pattern of Crystal Form III.
FIG. 5 Shows XRPD results of the active pharmaceutical ingredient in the formulation prepared using Crystal Form I (A); Transmission/Reflection XRPD comparison of Crystal Form I (B); Transmission/Reflection XRPD comparison of Crystal Form IIb (C).
FIG. 6 Shows XRPD results of the active pharmaceutical ingredient in the formulation prepared using Crystal Form IIb.
FIG. 7 shows cumulative permeation of drug in in vitro transdermal test.
FIG. 8 shows retention of drug in epidermis in in vitro transdermal test.
FIG. 9 shows retention of drug in dermis in in vitro transdermal test.
FIG. 10 shows cumulative permeation of Crystal Form I (A) and Crystal Form IIb (B) in rat transdermal test.

### Detailed Description

The following specific embodiments are provided to further illustrate the present disclosure, so that those skilled in the art can better understand the present disclosure and implement it. However, the examples described herein are not intended to limit the scope of the present disclosure.

### Preparation Examples

Topical gel formulations were prepared according to the compositions of each example.

### Examples 1 to 5:

| **Component** | **Example 1** | **Content** | **Example 2** | **Content** | **Function** |
|---|---|---|---|---|---|
| Crystal Form IIb | 300mg | 3.0% | 300mg | 3.0% | Active ingredient |
| Carbomer | 100mg | 1.0% | 100mg | 1.0% | Gel matrix |
| Propylene glycol (PG) | 1500mg | 15.0% | 2000mg | 20.0% | Solvent |
| PEG 400 | 750mg | 7.5% | 750mg | 7.5% | Solvent |
| Disodium edetate | 20mg | 0.2% | 20mg | 0.2% | Chelating agent |
| Methylparaben | 20mg | 0.2% | 20mg | 0.2% | Antimicrobial agent |
| Propylparaben | 2mg | 0.02% | 2mg | 0.02% | Antimicrobial agent |
| Sodium hydroxide | q.s. | - | q.s. | - | pH adjuster |
| Purified water | to 10g | to 100% | to 10g | to 100% | Solvent |

| **Component** | **Example 3** | **Content** | **Example 4** | **Content** | **Function** |
|---|---|---|---|---|---|
| Crystal Form IIb | 300mg | 3.0% | 300mg | 3.0% | Active ingredient |
| Carbomer | 100mg | 1.0% | 100mg | 1.0% | Gel matrix |
| Propylene glycol | 1500mg | 15.0% | 1500mg | 15.0% | Solvent |
| PEG 400 | 500mg | 5.0% | / | / | Solvent |
| Disodium edetate | 20mg | 0.2% | 20mg | 0.2% | Chelating agent |
| Methylparaben | 20mg | 0.2% | 20mg | 0.2% | Antimicrobial agent |
| Propylparaben | 2mg | 0.02% | 2mg | 0.02% | Antimicrobial agent |
| Sodium hydroxide | q.s. | - | q.s. | - | pH adjuster |
| Purified water | to 10g | to 100% | to 10g | to 100% | Solvent |

| **Component** | **Example 5** | **Content** | **Function** |
|---|---|---|---|
| Crystal Form IIb | 300mg | 3.0% | Active ingredient |
| Carbomer | 100mg | 1.0% | Gel matrix |
| PEG 400 | 500mg | 5.0% | Solvent |
| Disodium edetate | 20mg | 0.2% | Stabilizer |
| Methylparaben | 20mg | 0.2% | Antimicrobial agent |
| Propylparaben | 2mg | 0.02% | Antimicrobial agent |
| Sodium hydroxide | q.s. | - | pH adjuster |
| Purified water | to 10g | to 100% | Solvent |

### Preparation Process:

1. Phase I (Carbomer phase) preparation: Weigh an appropriate amount of purified water, slowly add carbomer, and constantly stirring until fully dispersed and swollen.
2. Phase II (Active ingredient phase) preparation: Weigh propylene glycol and PEG 400, add methylparaben and propylparaben and stir until completely dissolved; add Compound A, constantly stirring until uniformly dispersed, then transfer to a homogenizer.
3. Phase III (Disodium edetate phase) preparation: Weigh an appropriate amount of purified water, add disodium edetate and stir until completely dissolved, then transfer to the homogenizer.
4. Mixing and homogenization: Turn on the homogenizer, stirring and mixing for homogenization.
5. pH adjustment: Use sodium hydroxide solution to adjust pH to 5.0-7.0.
6. Vacuum stirring homogenization: Perform vacuum stirring and homogenization, then stop and discharge.

Examples 6-8 were prepared according to the preparation method of Examples 1-5.

### Example 6

| **Component** | **Weight/g** |
|---|---|
| Compound A (Crystal Form IIb) | 500mg |
| Carbomer | 100mg |
| Propylene glycol | 2500mg |
| PEG 400 | 500mg |
| Disodium edetate | 20mg |
| Methylparaben | 20mg |
| Propylparaben | 2mg |
| Sodium hydroxide | q.s. |
| Purified water | to 10g |

### Example 7

| **Component** | **Weight/g** |
|---|---|
| Compound A (Crystal Form IIb) | 150mg |
| Carbomer | 100mg |
| Propylene glycol | 750mg |
| PEG 400 | 500mg |
| Disodium edetate | 20mg |
| Methylparaben | 20mg |
| Propylparaben | 2mg |
| Sodium hydroxide | q.s. |
| Purified water | to 10g |

### Example 8

| **Component** | **Weight/g** |
|---|---|
| Compound A (Crystal Form IIb) | 50mg |
| Carbomer | 100mg |
| Propylene glycol | 500mg |
| PEG 400 | 500mg |
| Disodium edetate | 20mg |
| Methylparaben | 20mg |
| Propylparaben | 2mg |
| Sodium hydroxide | q.s. |
| Purified water | to 10g |

The gel formulation samples from Examples 3, 4, and 5 were evaluated for transdermal behavior and skin retention of the active ingredient in the formulation using 1-month-old Bama miniature pig isolated skin on Franz diffusion cells.

In vitro transdermal test results are as follows:
In vitro release cumulative permeation results

| **Permeation Time (h)** | Cumulative Permeation (ng/cm2) | | | | | |
|---|---|---|---|---|---|---|
| | **Example 3 (PG+PEG400)** | | **Example 4 (PG)** | | **Example 5 (PEG400)** | |
| | Mean | SD | Mean | SD | Mean | SD |
| 4 | 3.99 | 1.58 | 2.64 | 1.42 | 2.03 | 0.85 |
| 8 | 21.04 | 25.87 | 3.17 | 1.61 | 3.19 | 1.37 |
| 12 | 25.76 | 18.12 | 4.05 | 2.45 | 3.67 | 2.38 |
| 16 | 28.26 | 16.16 | 8.10 | 8.70 | 3.53 | 0.87 |
| 24 | 44.86 | 26.21 | 9.99 | 9.57 | 3.59 | 0.77 |

In vitro release intradermal retention results

| **Permeation Time (h)** | **Intradermal Retention (ng/cm²)** | | | |
|---|---|---|---|---|
| | Epidermis | | Dermis | |
| | Mean | SD | Mean | SD |
| Example 3 | 2.02 | 1.05 | 0.83 | 0.50 |
| Example 4 | 1.48 | 0.54 | 0.59 | 0.19 |
| Example 5 | 1.21 | 1.21 | 0.40 | 0.40 |

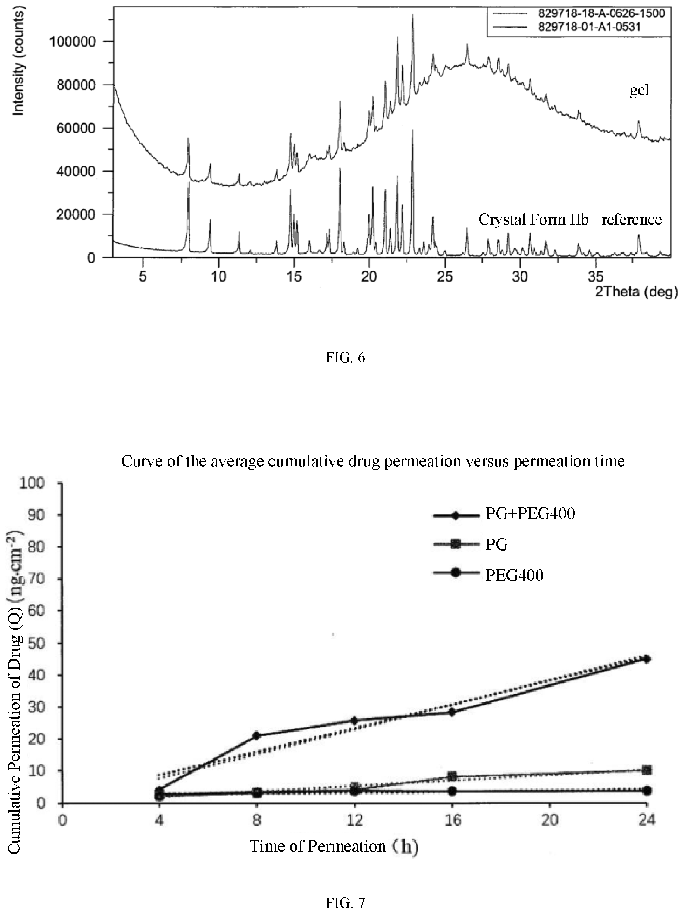

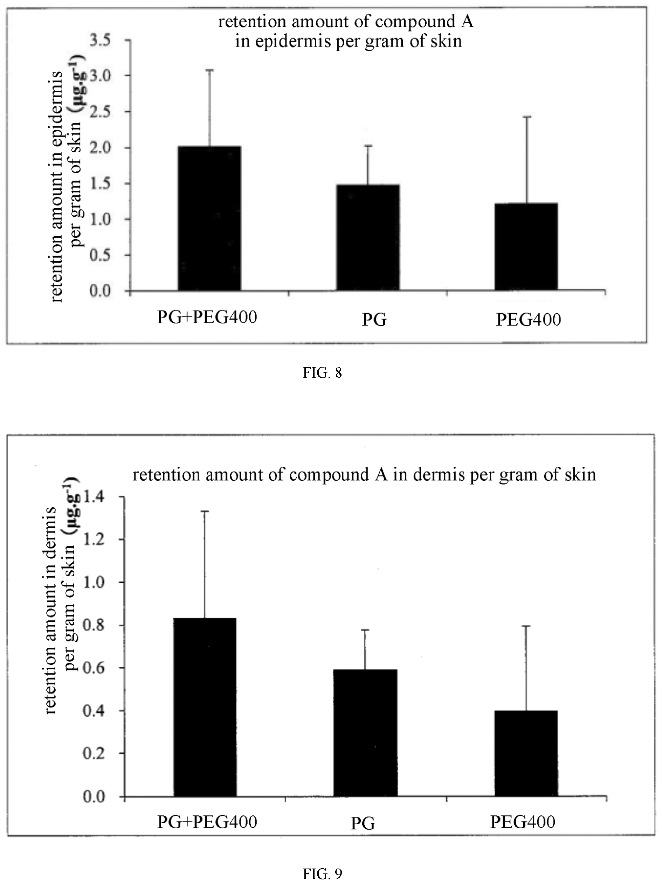

The above in vitro release cumulative permeation results are shown in FIG. 7, epidermis retention results are shown in FIG. 8, and dermis retention results are shown in FIG. 9.

Through in vitro transdermal testing, an unexpected discovery was made: compared to using propylene glycol or polyethylene glycol alone, the combined use of propylene glycol and polyethylene glycol increased drug permeation through skin by 4-11 times and increased intradermal epidermis and dermis retention by nearly 1 fold. The in vitro transdermal results indicate that the combined use of propylene glycol and polyethylene glycol can significantly improve the skin permeation ability of the active ingredient 2-{3-[3-amino-4-(7H-pyrrolo[2,3-d]pyrimidin-4-yl)-1H-pyrazol-1-yl]-1-(isopropylsulfonyl)azetidin-3-yl}acetonitrile.

### Example 9 Preparation of polymorphs of 2-{3-[3-amino-4-(7H-pyrrolo[2,3-d]pyrimidin-4-yl)-1H-pyrazol-1-yl]-1-(isopropylsulfonyl)azetidin-3-yl}acetonitrile

### A. Preparation of 2-{3-[3-amino-4-(7H-pyrrolo[2,3-d]pyrimidin-4-yl)-1H-pyrazol-1-yl]-1-(isopropylsulfonyl)azetidin-3-yl}acetonitrile Crystal Form I

To a 250 mL flask, add 20.0 g of 2-{ 3-[3-amino-4-(7H-pyrrolo[2,3-d]pyrimidin-4-yl)-1H-pyrazol-1-yl]-1-(isopropylsulfonyl)azetidin-3-yl}acetonitrile, 100 mL of N,N-dimethylformamide, stir to dissolve, filter to remove insoluble matter, add 250 mL of purified water to the filtrate, stir to crystallize, filter, transfer the obtained dried solid to a 250 mL flask, add 200 mL of anhydrous ethanol, heat to reflux for 3 hours, cool, filter, and dry the obtained solid at 55-60°C to constant weight to obtain 18.8 g of off-white solid, yield 94.0%, chemical purity 99.62%.

### B. Preparation of 2-{3-[3-amino-4-(7H-pyrrolo[2,3-d]pyrimidin-4-yl)-1H-pyrazol-1-yl]-1-(isopropylsulfonyl)azetidin-3-yl}acetonitrile Crystal Form IIa

To a 100 mL flask, add 5.0 g of 2-{3-[3-amino-4-(7H-pyrrolo[2,3-d]pyrimidin-4-yl)-1H-pyrazol-1-yl]-1-(isopropylsulfonyl)azetidin-3-yl}acetonitrile Crystal Form I, add 100 mL of ethanol (95%), heat and stir to reflux for 5 hours, cool, filter to obtain solid, dry the obtained solid under vacuum (0.1 MPa) at 80°C for 12 hours to obtain 4.8 g of solid, yield 96.0%, HPLC measured purity: 99.50%.

### C. Preparation of 2-{3-[3-amino-4-(7H-pyrrolo[2,3-d]pyrimidin-4-yl)-1H-pyrazol-1-yl]-1-(isopropylsulfonyl)azetidin-3-yl}acetonitrile Crystal Form IIb

To a 100 mL flask, add 6.2 g of 2-{3-[3-amino-4-(7H-pyrrolo[2,3-d]pyrimidin-4-yl)-1H-pyrazol-1-yl]-1-(isopropylsulfonyl)azetidin-3-yl}acetonitrile, 25 mL of N,N-dimethylformamide, stir to dissolve, filter to remove insoluble matter, add 50 mL of purified water to the filtrate, stir to crystallize, filter, and dry the obtained solid at 55-60°C to constant weight to obtain 5.6 g of solid, yield 88.9%.

### D. Preparation of 2-{3-[3-amino-4-(7H-pyrrolo[2,3-d]pyrimidin-4-yl)-1H-pyrazol-1-yl]-1-(isopropylsulfonyl)azetidin-3-yl}acetonitrile Crystal Form III

Add 15 mg of 2-{3-[3-amino-4-(7H-pyrrolo[2,3-d]pyrimidin-4-yl)-1H-pyrazol-1-yl]-1-(isopropylsulfonyl)azetidin-3-yl}acetonitrile to 3 mL of dioxane, heat to 40°C to dissolve and obtain a clear solution, filter, and allow the solution to crystallize by evaporation at 40°C to obtain Crystal Form III. This crystal form has poor crystallinity and poor stability, and after storage, NMR showed approximately 10% impurity formation.

### Solubility Study of Various Crystal Forms

### Solubility in 20% Propylene Glycol at 32°C (µg/mL)

| Solvent | Crystal Form I | Crystal Form IIb |
|---|---|---|
| 10 min | 12.7 | 6.1 |
| 30 min | 26.9 | 9.5 |
| 1 h | 35.8 | 10.8 |
| 2 h | 42.1 | 11.2 |
| 18 h | 20.2 | 13.3 |

### Solubility Measurement Results (37°C, 24 h)

| pH | Crystal Form I | Crystal Form IIb |
|---|---|---|
| | Conc. (mg/mL) | Conc. (mg/mL) |
| 1.0 | 0.4642 | 0.1499 |
| 2.0 | 0.0378 | 0.0174 |
| 3.8 | 0.0023 | 0.0009 |
| 4.5 | 0.0018 | 0.0007 |
| 5.5 | 0.0016 | 0.0005 |
| 6.8 | 0.0012 | 0.0006 |
| 7.0 | 0.0013 | 0.0008 |
| 8.0 | 0.0012 | 0.0007 |

### Stability Study of Various Crystal Forms

In accordance with the stability study guidelines issued by the China's National Medical Products Administration (NMPA), stability studies were conducted on Crystal Forms I, IIa, and IIb. Samples were packaged in pharmaceutical low-density polyethylene bags, and long-term stability (24 months) was evaluated. The results are as follows:

### Long-term 24-month stability of Crystal Form I

| **Time (month)** | **Appearance** | **Crystal Form** | **Loss on Drying (%)** | **Related Substances (%)** | | **Content (%)** |
|---|---|---|---|---|---|---|
| | | | | Max Unknown Single Impurity | Total Impurity | |
| 0 | Off-white powder | I | 0.08 | 0.17 | 0.58 | 99.9 |
| 3 | Off-white powder | I | 0.06 | 0.16 | 0.57 | 100.1 |
| 6 | Off-white powder | I | 0.08 | 0.17 | 0.57 | 100.0 |
| 9 | Off-white powder | I | 0.11 | 0.17 | 0.57 | 99.8 |
| 12 | Off-white powder | I | 0.09 | 0.17 | 0.55 | 99.9 |
| 18 | Off-white powder | I | 0.12 | 0.18 | 0.62 | 99.7 |
| 24 | Off-white powder | I | 0.10 | 0.16 | 0.59 | 99.8 |

### Long-term 24-month stability of Crystal Form IIa

| **Time (month)** | **Appearance** | **Crystal Form** | **Loss on Drying (%)** | **Related Substances (%)** | | **Content (%)** |
|---|---|---|---|---|---|---|
| | | | | Max Unknown Single Impurity | Total Impurity | |
| 0 | Off-white powder | IIa | 0.06 | 0.06 | 0.28 | 99.8 |
| 3 | Off-white powder | IIb | 0.18 | 0.08 | 0.28 | 97.6 |
| 6 | Off-white powder | IIb | 0.19 | 0.11 | 0.27 | 97.8 |
| 9 | Off-white powder | IIb | 0.24 | 0.06 | 0.24 | 97.9 |
| 12 | Off-white powder | IIb | 0.20 | 0.09 | 0.22 | 97.8 |
| 18 | Off-white powder | IIb | 0.16 | 0.07 | 0.26 | 98.0 |
| 24 | Off-white powder | IIb | 0.25 | 0.08 | 0.29 | 97.8 |

### Long-term 24-month stability of Crystal Form IIb

| **Time (month)** | **Appearance** | **Crystal Form** | **Loss on Drying (%)** | **Related Substances (%)** | | **Content (%)** |
|---|---|---|---|---|---|---|
| | | | | Max Unknown Single Imp. | Total Imp. | |
| 0 | Off-white powder | IIb | 0.36 | 0.07 | 0.38 | 99.7 |
| 3 | Off-white powder | IIb | 0.36 | 0.08 | 0.38 | 99.7 |
| 6 | Off-white powder | IIb | 0.39 | 0.08 | 0.37 | 99.6 |
| 9 | Off-white powder | IIb | 0.34 | 0.08 | 0.38 | 99.5 |
| 12 | Off-white powder | IIb | 0.30 | 0.07 | 0.37 | 99.6 |
| 18 | Off-white powder | IIb | 0.31 | 0.07 | 0.39 | 99.6 |
| 24 | Off-white powder | IIb | 0.32 | 0.08 | 0.39 | 99.7 |

The above stability study results indicate that Crystal Forms I and IIb of 2-{3-[3-amino-4-(7H-pyrrolo[2,3-d]pyrimidin-4-yl)-1H-pyrazol-1-yl]-1-(isopropylsulfonyl)azetidin-3-yl}acetonitrile have good chemical stability. Crystal Form IIa gradually absorbs moisture during storage and converts to Crystal Form IIb, with sample weight gain due to water absorption, resulting in decreased content.

### Transdermal Test of Crystal Forms

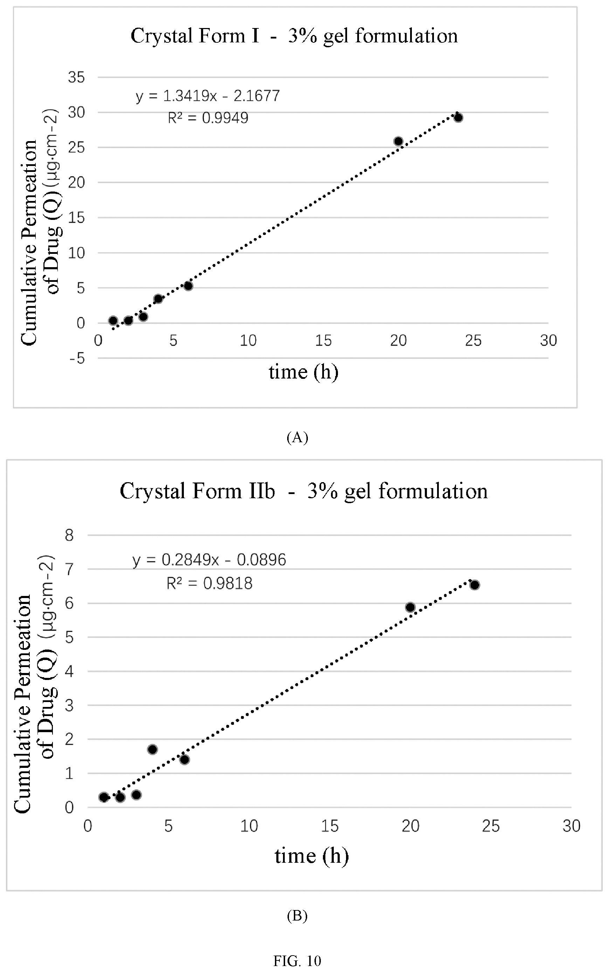

Crystal Forms I and IIb were prepared into topical gel formulations according to the compositions of Example 1, and transdermal tests were immediately conducted to determine their respective transdermal rates. The test results are shown in FIG. 10A and FIG. 10B, indicating that Crystal Form I has better transdermal performance.

Blood samples were collected at 0.25, 0.5, 1, 2, 4, 6, 8, and 24 hours after topical administration. Using a small animal anesthesia machine, 0.3 mL of whole blood was collected from the retro-orbital venous plexus under isoflurane anesthesia and placed in heparin anticoagulant tubes. Samples were centrifuged at 4°C, 4000 rpm for 5 minutes, plasma was transferred to centrifuge tubes, and stored at -80°C until analysis. Samples in plasma were extracted using protein precipitation, and the extract was analyzed by LC/MS/MS. After the test, the drug on skin was washed off, and the rat skin at administration site was excised, homogenized, and analyzed by LC/MS/MS for drug content in the skin.

**Table 4 Drug Concentration in Skin 24 h After Topical Administration**

| | **Crystal Form I, 3% Gel** | **Crystal Form IIb, 3% Gel** |
|---|---|---|
| Average Drug Concentration in Rat Skin (µg/g) | 91.4 | 579.5 |

Crystal Forms I and IIb have the characteristics of high crystallinity and good stability. Crystal Form I has higher solubility than Crystal Form IIb. Although Crystal Form IIb has inferior transdermal absorption compared to Crystal Form I, its topical formulation shows significantly higher drug retention concentration in animal skin than Crystal Form I by several fold. This unexpected characteristic makes Crystal Form IIb more suitable for topical gel formulations with less systemic penetration but higher drug retention concentration in the skin.

## Claims

1. A topical gel formulation comprising:
(a) 2-{3-[3-amino-4-(7H-pyrrolo[2,3-d]pyrimidin-4-yl)-1H-pyrazol-1-yl]-1-(isopropylsulfonyl)azetidin-3-yl}acetonitrile, its hydrate, its pharmaceutically acceptable salt, or its crystal form;
(b) a gel matrix;
(c) propylene glycol in an amount of 5% to 30% by total weight of the topical gel formulation; and
(d) polyethylene glycol in an amount of 2% to 20% by total weight of the topical gel formulation.

2. The topical gel formulation according to claim **1,** wherein the gel matrix is selected from carbomer.

3. The topical gel formulation according to claim 2, wherein carbomer is in an amount of 0.8% to 1.5% by total weight of the topical gel formulation.

4. A topical gel formulation comprising:
(a) 2-{3-[3-amino-4-(7H-pyrrolo[2,3-d]pyrimidin-4-yl)-1H-pyrazol-1-yl]-1-(isopropylsulfonyl)azetidin-3-yl}acetonitrile hemihydrate crystal form, wherein the characteristic peaks in the powder X-ray diffraction pattern have 2θ values of 7.93, 9.36, 11.29, 14.95, 20.96, 21.36, 21.77, 22.12, and 22.79, with a measurement error of ±0.20 degrees.

5. The topical gel formulation according to claim 4, further comprising:
(b) a gel matrix; and
(c) propylene glycol in an amount of 5% to 30% by total weight of the topical gel formulation.

6. The topical gel formulation according to claim 4 or 5, further comprising polyethylene glycol.

7. The topical gel formulation according to claim 6, wherein the polyethylene glycol is in an amount of 2% to 20% by total weight of the topical gel formulation.

8. The topical gel formulation according to any one of claims 4-7, wherein the gel matrix is selected from carbomer.

9. The topical gel formulation according to claim 8, wherein the carbomer is an amount of 0.8% to 1.5% by total weight of the topical gel formulation.

10. A topical gel formulation comprising:
(a) 2-{ 3-[3-amino-4-(7H-pyrrolo[2,3-d]pyrimidin-4-yl)-1H-pyrazol-1-yl]-1-(isopropylsulfonyl)azetidin-3-yl}acetonitrile hemihydrate crystal form, wherein the characteristic peaks in the powder X-ray diffraction pattern have 2θ values of 7.93, 9.36, 11.29, 14.95, 20.96, 21.36, 21.77, 22.12, and 22.79, with a measurement error of ±0.2 degrees; and
(b) a gel matrix, which is carbomer in an amount of 0.8% to 1.5% by total weight of the topical gel formulation.

11. The topical gel formulation according to claim 10, further comprising polyethylene glycol and propylene glycol.

12. The topical gel formulation according to claim 11, wherein the propylene glycol is in an amount of 5% to 30% by total weight of the topical gel formulation; and the polyethylene glycol is in an amount of 2% to 20% by total weight of the topical gel formulation.

13. The topical gel formulation according to any one of claims 1-12, wherein the polyethylene glycol has average molecular weight of 200 to 600.

14. The topical gel formulation according to any one of claims 1-13, further comprising one or more of the following pharmaceutical excipients: antimicrobial agents, antioxidants, and pH adjusters.

15. Use of the topical gel formulation according to any one of claims 1-14 in the preparation of a drug for inhibiting JAK.
